# EUROPEAN PATENT SPECIFICATION

(11) **EP 3 510 168 B1**
(45) Date of publication and mention of the grant of the patent: **08.07.2020**
(21) Application number: 17765145.2
(22) Date of filing: 06.09.2017
(51) Int. Cl.: C12Q 1/6818, C12Q 1/6823, C12Q 1/6869

(54) **SINGLE NUCLEOTIDE DETECTION METHOD AND ASSOCIATED PROBES**
EINZELNUKLEOTIDDETEKTIONSVERFAHREN UND ZUGEHÖRIGE SONDEN
PROCÉDÉ DE DÉTECTION D'UN NUCLÉOTIDE SIMPLE ET SONDES ASSOCIÉES

(30) Priority: 06.09.2016 EP 16187493; 09.11.2016 GB 201618918
(43) Date of publication of application: 17.07.2019
(73) Proprietor: Base4 Innovation Limited, Cambridge, Cambridgeshire CB3 0FA (GB)
(72) Inventor: BALMFORTH, Barnaby, Cambridge Cambridgeshire CB3 0FA (GB); FRAYLING, Cameron Alexander, Cambridge Cambridgeshire CB3 0FA (GB)
(74) Representative: Lau, Sarah Jane
(86) International application number: PCT/EP2017/072308
(87) International publication number: WO 2018/046521

(56) References cited:
- EP-A2- 0 878 554
- WO-A1-2014/053853
- WO-A1-2016/012789
- WO-A2-2006/088911
- CUI WANLING ET AL: "A dual amplification fluorescent strategy for sensitive detection of DNA methyltransferase activity based on strand displacement amplification and DNAzyme amplification", BIOSENSORS AND BIOELECTRONICS, vol. 77, 22 October 2015 (2015-10-22), pages 650-655, XP029311813, ISSN: 0956-5663, DOI: 10.1016/J.BIOS.2015.10.040

## Description

This invention relates to a method and associated biological probe systems for detecting and characterising single nucleotides. It is especially suitable for use in the sequencing of DNA or RNA.

Next generation sequencing of genetic material is already making a significant impact on the biological sciences in general and medicine in particular as the unit cost of sequencing falls in line with the coming to market of faster and faster sequencing machines.

In our previous applications WO 2014/053853, WO 2014/053854, WO2014/167323, WO2014/167324 and WO2014/111723 we have described a new sequencing method which involves progressive digestion of a polynucleotide analyte to generate an ordered stream of single nucleotides, preferably a stream of single nucleoside triphosphates, each of which can be captured one-by-one into corresponding droplets in a microdroplet stream. Thereafter, each droplet can be chemically and/or enzymatically manipulated to reveal the particular single nucleotide it originally contained. In one embodiment, these chemical and/or enzymatic manipulations comprise a method involving the use of one or more two-component oligonucleotide probe types each of which is adapted to be able to selectively capture one of the single nucleotide types from which the analyte is constituted. Typically, in each of such probe types, one of the two oligonucleotide components comprises characteristic fluorophores and in the probe's unused state the ability of these fluorophores to fluoresce remains extinguished by virtue of the presence of quenchers located close-by or by self-quenching. In use, when the probe has captured its corresponding single nucleotide, it is rendered susceptible to subsequent exonucleolysis thereby liberating the fluorophores from the quenchers and/or each other enabling them to fluoresce freely. By this means, the original single nucleotide present in each droplet can be inferred indirectly by spectroscopic means.

Variants of this method have been described in other of our pending applications including WO201405385 and WO2016012789; the latter involving the use of a three-component probe system. In particular, WO2016012789 describes an improved method characterised by the steps of (1) generating a stream of single nucleoside triphosphates by progressive digestion of the nucleic acid; (2) producing at least one substantially double-stranded oligonucleotide used probe by reacting in the presence of a polymerase and a ligase at least one of the single nucleoside triphosphates with a corresponding probe system comprising (a) a first single-stranded oligonucleotide labelled with e.g. characteristic fluorophores in an undetectable state and (b) second and third single-stranded oligonucleotides capable of hybridising to complementary regions on the first oligonucleotide; (3) digesting the used probe with an enzyme having double-stranded exonucleolytic activity to yield the fluorophores in a detectable state and a single-stranded fourth oligonucleotide which is at least in part the sequence complement of the first oligonucleotide; (4) reacting the fourth oligonucleotide with another first oligonucleotide to produce a substantially double-stranded oligonucleotide product corresponding to the used probe; (5) repeating steps (3) and (4) in a cycle and (6) detecting the fluorophores released in each iteration of step (3). This method has the advantage that by iterating steps (3) and (4) in a cycle the fluorescence signal can be made to grow strongly thereby improving the overall sensitivity and therefore reliability of nucleotide detection. In one embodiment, the second and third oligonucleotides are linked so that, after nucleotide capture, they form a closed-loop single-stranded oligonucleotide component which is advantageously resistant to exonucleolysis.

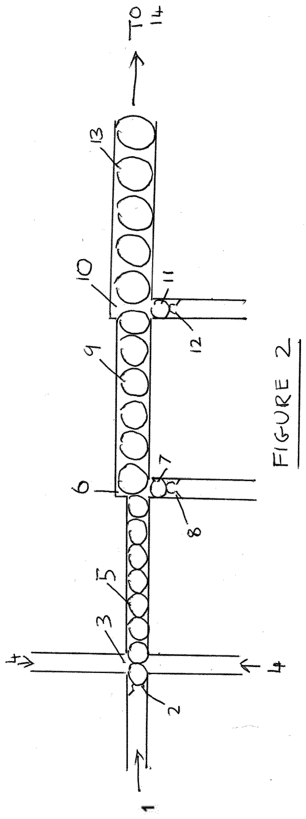

As regards other prior art, Fan et al in Nature Reviews Genetics 7(8) 632-644 (2006) provide a general review of the development of methods and platforms that have enabled highly parallel genomic assays for genotyping, copy-number measurements, sequencing and detecting loss of heterozygosity, allele-specific expression and methylation. Figure 2a of this review schematically shows the use of a circularizable probe with 3' and 5' ends that anneal upstream and downstream of a site of single nucleotide polymorphism (SNP) on an analyte thereby leaving a gap which is subsequently filled with a nucleotide which is the complement of the SNP to form a complete circular probe which may then be amplified after release. However, unlike our method, the nucleotide which is captured during the filling process is not obtained directly from the analyte itself.

WO03080861 discloses a process wherein a nucleic acid analyte is subjected to progressive pyrophosphorolysis in the presence of a nucleotide-specific reactive label which attaches directly to the nucleotide as it is released. Not only is this quite different from the method we employ but in practice the fluorescence signal measured when the labelled nucleotides are subsequently interrogated would likely be too weak to enable reliable identification above the associated background noise.

Finally, WO9418218 teaches a DNA sequencing method in which the analyte is subjected to progressive exonucleolysis to generate a stream of single nucleotide diphosphates or monophosphates which are then incorporated into a fluorescence-enhancing matrix before being detected. Not only is this a completely different approach to the one we describe but we again observe that any signal generated would likely be too weak to be reliably detected and identified.

We have now invented an improved method of generating an even stronger fluorescence signal which is suitable for use with the droplet-based sequencers we have previously described. Thus, according to the present invention there is provided a method of sequencing a nucleic acid characterised by the steps of (1) generating a stream of single nucleoside triphosphates by progressive enzymatic digestion of the nucleic acid; (2) producing at least one substantially double-stranded primary oligonucleotide used probe by reacting, in the presence of a polymerase and a ligase, at least one of the single nucleoside triphosphates with a corresponding primary probe comprising (a) a first single-stranded oligonucleotide including a single nucleotide capture site for capturing the single nucleoside triphosphate, a restriction endonuclease nicking site including the capture site and oligonucleotide flanking regions juxtaposed either side of the capture site and (b) second and third single-stranded oligonucleotides capable of hybridising to the first oligonucleotide flanking regions; (3) nicking the first oligonucleotide strand of the used primary probe at the nicking-site with a nicking restriction endonuclease to create separate first oligonucleotide components; (4) separating the first oligonucleotide components generated in step (3) from the complementary strand of the used probe; (5) producing at least one substantially double-stranded secondary used probe by reacting, in the presence of a ligase, at least one of the separated first oligonucleotide components with a corresponding secondary probe comprising (c) a fourth oligonucleotide having an oligonucleotide region which is complementary to a corresponding region of the first oligonucleotide component and bearing fluorophores in a substantially undetectable state and optionally (d) a single-stranded fifth oligonucleotide having an oligonucleotide region which is at least in part complementary to another corresponding region of the fourth oligonucleotide to produce a double-stranded used secondary probe comprised of the first oligonucleotide component, the fourth oligonucleotide and optionally the fifth oligonucleotide; (6) digesting the fourth oligonucleotide strand of the used secondary probe with an enzyme having double-stranded exonucleolytic activity to yield single nucleotides bearing fluorophores in a detectable state and (7) detecting the fluorophores released in step (6).

In one preferred embodiment, the complementary strand of the used probe released in step (4) is reacted with a further molecule of the first oligonucleotide and steps (3) and (4) are thus iterated in a first cycle to increase significantly the number of fluorophores released for detection in step (7). In another embodiment the sixth oligonucleotide generated in step (6) is reacted with a further molecule of the fourth oligonucleotide and step (6) thereafter repeated to create a second cycle Preferably the method of the invention involves iteration using both first and second cycles.

Step (1) of the method of the present invention comprises generating a stream of single nucleoside triphosphates from a nucleic acid analyte by progressive enzymatic digestion. In one embodiment this can be achieved by progressive exonucleolysis of the analyte followed by the action of a kinase on the single nucleoside monophosphates obtained (see for example Bao and Ryu, Biotechnology and Bioengineering DOI 10.1002/bit (May 2007)). In another embodiment step (1) comprises generating a stream of single nucleoside triphosphates directly from the analyte by progressive pyrophosphorolysis. The analyte employed in this step is suitably a double-stranded polynucleotide the length of which can in principle be unlimited; for example, including up to the many millions of nucleotide pairs found in a human gene or chromosome fragment. Typically, however, the polynucleotide will be at least 50, preferably at least 150 nucleotide pairs long; suitably it will be greater than 500, greater than 1000 and in many cases thousands of nucleotide pairs long. The analyte itself is preferably RNA or DNA of natural origin (e.g. derived from a plant, animal, bacterium or a virus) although the method can also be used to sequence synthetically-produced RNA or DNA or other nucleic acids made up wholly or in part of nucleotides whose nucleobases are not commonly encountered in nature; i.e. nucleobases other than adenine (A), guanine (G), cytosine (C), thymine (T) and uracil (U). Examples of such nucleobases include 4-acetylcytidine, 5-(carboxyhydroxylmethyl)uridine, 2-O-methylcytidine, 5-carboxymethylaminomethyl-2-thiouridine, 5-carboxymethylamino-methyluridine, dihydrouridine, 2-O-methylpseudouridine, 2-O-methylguanosine, inosine, N6-isopentyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, 5-methoxyuridine, 5-methoxycarbonylmethyl-2-thiouridine, 5-methoxycarbonylmethyluridine, 2-methylthio-N6-isopentenyladenosine, uridine-5-oxyacetic acid-methylester, uridine-5-oxyacetic acid, wybutoxosine, wybutosine, pseudouridine, queuosine, 2-thiocytidine, 5-methyl-2-thiouridine, 2-thiouridine, 4-thiouridine, 5-methyluridine, 2-O-methyl-5-methyluridine and 2-O-methyluridine. In the case of DNA, the single nucleoside triphosphates generated are deoxyribonucleoside triphosphates whilst in the case of RNA they are ribonucleoside triphosphates.

In one embodiment of the method, step (1) further comprises a first sub-step of attaching the analyte to a substrate. Typically, this substrate comprises a microfluidic surface, a micro-bead or a permeable membrane; for example, one made of glass or a non-degradable polymer. Preferably, the substrate further comprises a surface specifically adapted to receive the analyte. There are many ways in which the analyte can be attached to such surfaces any of which can in principle be used in this sub-step. For example, one method involves priming a glass surface with a functionalised silane such as an epoxysilane, an aminohydrocarbylsilane or a mercaptosilane. The reactive sites so generated can then be treated with a derivative of the analyte which has been modified to include a terminal amine, succinyl or thiol group.

In another embodiment of step (1), the analyte is pyrophosphorolysed to generate a stream of single nucleoside triphosphates the order of which corresponds to that of the sequence of the analyte. Such pyrophosphorolysis may be carried out at a temperature in the range 20 to 90°C; for example, in the presence of a reaction medium comprising a suitable polymerase. Preferably it is carried out under conditions of continuous flow so that the single nucleoside triphosphates are continually removed from the reaction zone as they are liberated. Most preferably, the pyrophosphorolysis is carried out by causing an aqueous buffered medium containing the enzyme and the other typical additives to continuously flow over the surface to which the analyte is bound.

In yet another embodiment, the enzyme used is one which can cause progressive 3'-5' pyrophosphorolytic degradation of the analyte to yield a stream of nucleoside triphosphates with high fidelity and at a reasonable reaction rate. Preferably this degradation rate is as fast as possible and in one embodiment is in the range 1 to 50 nucleoside triphosphates per second.

Further information about the pyrophosphorolysis reaction as applied to the digestion of polynucleotides can be found for example in J. Biol. Chem. 244 (1969) pp. 3019-3028. Suitably, the pyrophosphorolytic digestion is carried out in the presence of a medium which further comprises pyrophosphate anion and magnesium cations; preferably in millimolar concentrations.

In step (2) of the method of the present invention at least one single nucleoside triphosphate, preferably each single nucleoside triphosphate in the stream, is reacted in the presence of a polymerase and a ligase with a primary probe to generate a substantially double-stranded used primary probe. Preferably, before this step is carried out the product of step (1) is treated with a pyrophosphatase, to hydrolyse any residual pyrophosphate to phosphate anion.

Examples of polymerases which can be advantageously used include, but are not limited to, the prokaryotic pol 1 enzymes or enzyme derivatives obtained from bacteria such as *Escherichia coli* (e.g. Klenow fragment polymerase), *Thermus aquaticus* (e.g. *Taq* Pol), *Bacillus stearothermophilus, Bacillus caldovelox* and *Bacillus caldotenax.* Any suitable ligase can in principle be used in this step.

Each primary probe employed in step (2) is suitably comprised of (a) a first single-stranded oligonucleotide including a restriction endonuclease nicking-site, a single nucleotide capture site for capturing the nucleoside triphosphate and oligonucleotide flanking regions juxtaposed either side of the capture site and (b) second and third single-stranded oligonucleotides capable of hybridising to the flanking regions. In one embodiment, the second and third oligonucleotides are discrete entities whilst in another they are linked to each other by means of a linker region. In this latter case and in one embodiment, the linker region links ends of the second and third oligonucleotides. The linker region can in principle be any divalent group but is conveniently another oligonucleotide region. In one embodiment this oligonucleotide linker region is unable to hybridise substantially to the first oligonucleotide.

The first, second and third oligonucleotides are chosen so that in step (2) the second and third oligonucleotides hybridise respectively to 3' side and 5' side flanking regions on the first oligonucleotide juxtaposed either side of a capture site. In one embodiment, there is a nucleotide mismatch between the 3' end of the third oligonucleotide and the corresponding nucleotide on the 5' side of the first oligonucleotide. In another embodiment the 3' end of the third oligonucleotide also includes an exonucleolysis blocking group. The capture site comprises the single nucleotide whose nucleobase is complementary to the one borne by the nucleoside triphosphate to be detected. This makes the three-component primary probe highly selective for that particular nucleoside triphosphate. For example, if the analyte is derived from DNA and the first, second and third oligonucleotides are comprised of deoxyribonucleotides, the capture site will be highly selective for deoxyadenosine triphosphate if the nucleotide it comprises bears a thymine nucleobase. Thus, in one useful embodiment of the invention step (2) may be carried out in the presence of a probe system comprised of a plurality of primary probe types; for example, one, two, three or four primary probe types each of which comprises a first oligonucleotide having different flanking regions and a different capture site characteristic of the various different nucleobases sought.

Typically, the first oligonucleotide is up to 150 nucleotides long, preferably between 10 and 100 nucleotides. In one embodiment the second oligonucleotide is shorter than the complementary 3' side flanking region of the first oligonucleotide by at least one nucleotide. In another, there is a single nucleotide mismatch between the 3' end of the first oligonucleotide and the nucleotide opposite it on the second oligonucleotide to prevent the nucleoside triphosphate being captured by the polymerase at this point. Similarly, in one embodiment the third oligonucleotide is longer than the complementary 5' side flanking region of the first oligonucleotide by at least one nucleotide, while in another there is a single nucleotide mismatch between the 3' end of the third oligonucleotide and the nucleotide opposite it in the first oligonucleotide to prevent the single nucleoside triphosphate being captured by the polymerase at this point.

The nicking restriction endonuclease recognition site comprises an oligonucleotide region on the first oligonucleotide which itself includes the capture site. In another embodiment, where more than one primary probe type is employed, it is preferred that each first oligonucleotide nevertheless comprises a common nicking restriction endonuclease recognition site so that only one nicking restriction endonuclease need be employed. To achieve this, it is therefore preferred that the recognition site will be comprised of a sequence containing at least one of each of the typical nucleotides of RNA or DNA as the case may be.

In another preferred embodiment, the nicking restriction endonuclease as defined herein is a conventional restriction endonuclease otherwise capable of cleaving both strands of the used primary probe and in the region of the nicking-site the strand created during the primary probe's use is rendered resistant to endonucleolysis; for example, by including one or more endonucleolytic blocking sites in the second and/or third oligonucleotides. In one embodiment, these blocking groups may be selected from phosphorothioate linkages and other backbone modifications commonly used in the art, C3 spacers, phosphate groups, or the like.

Step (2) is suitably carried out by contacting each single nucleoside triphosphate in the stream with the enzymes and one or more primary probe types described above at a temperature in the range 20 to 80°C.

The product of step (2) of the method of the invention is, as mentioned above, a substantially double-stranded used primary probe whose constituent strands are respectively the first oligonucleotide and a complementary oligonucleotide comprised of the second oligonucleotide, a nucleotide derived from the single nucleoside triphosphate and finally the third oligonucleotide. If the second and third oligonucleotides have previously been joined together by a linker region, then it will be readily apparent that this complimentary oligonucleotide will comprise a closed-loop strand.

In step (3) the used primary probe is treated with a nicking restriction endonuclease at a temperature in the range 20 to 100°C. In this step the strand of the used primary probe derived from the first oligonucleotide is severed into two separate oligonucleotide components which can then in step (4) be de-hybridised from the probe's complementary strand thereby separating them from each other. Step (4) can be achieved by heating the nicked, used probe to a temperature in the range 30 to 100°C, but is most preferably achieved at the same temperature as that used in step (3).

In step (5) at least one of the separated oligonucleotide components is reacted in the presence of a ligase and a corresponding secondary probe to create at least one substantially double-stranded secondary used probe. In one embodiment this secondary probe is comprised of a partially double-stranded fourth oligonucleotide bearing fluorophores in an undetectable state and which is at least in part comprised of a single-stranded region which is the sequence complement of the relevant separated oligonucleotide component. In another, this fourth oligonucleotide is j-shaped as described in our application WO2014167323 to which the reader is directed for further information. In yet another embodiment the secondary probe comprises two components; (c) a single-stranded fourth oligonucleotide bearing fluorophores in an undetectable state and which is at least in part comprised of a single-stranded region which is the sequence of the separated oligonucleotide component and (d) a single-stranded fifth oligonucleotide which is at least in part comprised of a single-stranded region which is the sequence complement of at least part of the fourth oligonucleotide. Suitably the relevant oligonucleotide region and/or the fifth oligonucleotide include an element resistant to exonucleolytic degradation.

It is a feature of the fourth oligonucleotide that it is provided with a region which is multiply-labelled with its own unique type of fluorophores and that these fluorophores are arranged so as to be substantially undetectable when the secondary probe is in an unused state. Preferably these fluorophores are arranged to be essentially non-fluorescing at those wavelengths where they are designed to be detected. Thus, although a fluorophore may exhibit general, low-level background fluorescence across a wide part of the electromagnetic spectrum, there will typically be one or a small number of specific wavelengths or wavelength envelopes where the intensity of the fluorescence is at a maximum. It is at one or more of these maxima where the fluorophore is characteristically detected that essentially no fluorescence should occur. In the context of this patent, by the term 'essentially non-fluorescing' or equivalent wording is meant that the intensity of fluorescence of the total number of fluorophores attached to the fourth oligonucleotide at the relevant characteristic wavelength or wavelength envelope is less than 25%; preferably less than 10%; more preferably less than 1% and most preferably less than 0.1% of the corresponding intensity of fluorescence of an equivalent number of free fluorophores.

In principle, any method can be used to ensure that in the fourth oligonucleotide's unused state the fluorophores are essentially non-fluorescing. In one embodiment this is achieved by disposing the fluorophores in close proximity to quenchers. In another, it is achieved by arranging multiple fluorophores in close proximity to each other so that they tend to quench each other sufficiently well that quenchers are not required. In this context of this patent, what constitutes 'close proximity' between fluorophores or between fluorophores and quenchers will depend on the particular fluorophores and quenchers used and possibly the structural characteristics of the fourth oligonucleotide. Consequently, it is intended that this term should be construed with reference to the required outcome rather than any particular structural arrangement of these various elements. However, and for the purposes of providing exemplification only, it is pointed out that when adjacent fluorophores or adjacent fluorophores and quenchers are separated by a distance corresponding to the characteristic Forster distance (typically less than 5nm) sufficient quenching will generally be achieved.

As regards the fluorophores themselves, they can in principle be chosen from any of those conventionally used in the art including but not limited to xanthene moieties e.g. fluorescein, rhodamine and their derivatives such as fluorescein isothiocyanate, rhodamine B and the like; coumarin moieties (e.g. hydroxy-, methyl- and aminocoumarin) and cyanine moieties such as Cy2, Cy3, Cy5 and Cy7. Specific examples include fluorophores derived from the following commonly used dyes: Alexa dyes, cyanine dyes, Atto Tec dyes, and rhodamine dyes. Examples also include: Atto 633 (ATTO-TEC GmbH), Texas Red™, Atto 740 (ATTO-TEC GmbH), Rose Bengal, Alexa Fluor™ 750 C₅-maleimide (Invitrogen), Alexa Fluor™ 532 C₂-maleimide (Invitrogen) and Rhodamine Red C₂-maleimide and Rhodamine Green as well as phosphoramadite dyes such as Quasar 570. Alternatively, a quantum dot or a near infra-red dye such as those supplied by LI-COR Biosciences can be employed. The fluorophore is typically attached to the fourth oligonucleotide via a nucleobase using chemical methods known in the art.

Suitable quenchers include those which work by a Forster resonance energy transfer (FRET) mechanism. Examples of commercially available quenchers which can be used in association with the above mentioned-fluorophores include but are not limited to DDQ-1, Dabcyl, Eclipse, Iowa Black FQ and RQ, IR Dye-QC1, BHQ-0, BHQ-1, -2 and -3 and QSY-7 and -21.

In one embodiment the flanking regions of the first oligonucleotide also further comprises fluorophores as described above arranged so that the first oligonucleotide is substantially non-fluorescing in its unused state. In another embodiment at least one of the flanking regions further comprises quenchers.

The product of step (5) of the method of the invention is a substantially double-stranded used secondary probe comprised of the fourth oligonucleotide, the relevant first oligonucleotide component and optionally the fifth oligonucleotide. In one embodiment there is suitably a mismatch between the nucleotide at the 3' end of the fourth oligonucleotide and the corresponding nucleotide on the relevant first oligonucleotide component to prevent nucleotide capture at this site. In another embodiment there is suitably a mismatch between the nucleotide at the 3' end of the fifth oligonucleotide and the corresponding nucleotide on the fourth oligonucleotide to prevent nucleotide capture at this site.

Thereafter in step (6) the used secondary probe is digested with an enzyme having exonucleolytic activity at a temperature in the range 30 to 100°C. In this step the part of the used secondary probe derived from the fourth oligonucleotide is digested into its constituent nucleotides in the process; separating the fluorophores from one another and thereby causing them to become unquenched and therefore detectable. Thus as exonucleolysis proceeds, the observer sees a rapid growth in the fluorescence signal as a cascade of single nucleotides is generated. The characteristics of this fluorescence then indirectly reflects the nature of the single nucleoside triphosphate originally captured by the primary probe.

Enzymes which can be used in step (6) include Q5, Q5 Hot Start, Phusion, Phusion HS, Phusion II, Phusion II HS, Dnase I (RNase-free), Exonuclease I or III (ex. *E. coli*), Exonuclease T, Exonuclease V (RecBCD), Lambda Exonuclease, Micrococcal Nuclease, Mung Bean Nuclease, Nuclease BAL-31, RecJ_{f}, T5 Exonuclease and T7 Exonuclease.

It will be appreciated that steps (3) and (4) can be iterated in a first cycle by allowing a further first oligonucleotide to anneal to the complementary strand of the used probe subsequent to step (4), thus allowing the build-up of a high concentration of the relevant separated oligonucleotide components thereby allowing a corresponding high concentration of used secondary probes to be created. Also in another embodiment the sixth oligonucleotide generated in step (6) may be reacted with a further molecule of the fourth oligonucleotide and step (6) thereafter repeated to create a second cycle. Thus, when both the first and second cycles are iterated at the same time a double multiplier effect may be obtained. By this means, the fluorescence signal can be made to grow very quickly to a high intensity making its detection and identification easy.

Thereafter, and in step (7), the fluorophores liberated in step (6) are detected and the nature of the nucleobase attached to the single nucleoside triphosphate determined by inference. By carrying out the method of the invention systematically for all the single nucleoside triphosphates in an ordered stream generated in step (1), data characteristic of the sequence of original nucleic acid analyte can be generated and analysed. Methods of doing this are well-known in the art; for example, the reaction medium can be interrogated with light from a laser and any fluorescence generated detected using a photodetector or an equivalent device tuned to the characteristic fluorescence wavelength(s) or wavelength envelope(s) of the various fluorophores. This in turn causes the photodetector to generate a characteristic electrical signal which can be processed and analysed in a computer using known algorithms.

In one particularly preferred embodiment, the method of the present invention is carried out wholly or partially in a stream of microdroplets, at least some of which contain a single nucleoside triphosphate; suitably an ordered stream. Such a method may begin, for example, by inserting the nucleoside triphosphates generated in step (1) one-by-one into a corresponding stream of aqueous microdroplets maintained in an immiscible carrier solvent such as a hydrocarbon or silicone oil to help preserve the ordering. Alternatively, this can be achieved by directly creating the microdroplets downstream of the digestion (pyrophosphorolysis) zone; for example, by causing the reaction medium to emerge from a microdroplet head of suitable dimensions into a flowing stream of the solvent. Alternatively, small aliquots of the reaction medium from step (1) can be regularly and sequentially injected into a stream of pre-existing aqueous microdroplets suspended in the solvent. If this latter approach is adopted, each microdroplet may already contain the various components of the primary and secondary probes together with the enzymes and any other reagents (e.g. buffer) required to effect steps (2) to (6). In yet another approach, the microdroplets created in the former embodiment can be caused to coalesce subsequently with a stream of such pre-existing microdroplets to achieve a similar outcome. In these microdroplet methods, step (7) then preferably involves delivering the microdroplets to a storage area and interrogating each microdroplet to identify the fluorophores liberated. Thereafter the results obtained from each microdroplet are assembled into a stream of data characteristic of the sequence of the original nucleic acid analyte.

To avoid the risk that a given microdroplet contains more than one nucleoside triphosphate it is preferred to release each nucleoside triphosphate in step (1) at a rate such that each filled microdroplet is separated on average by from 1 to 20 preferably 2 to 10 empty ones. Thereafter the stream of filled and unfilled microdroplets in the solvent is caused to flow along a flow path, suitably a microfluidic flow path, at a rate and in a manner such that they are maintained in a discrete state and do not have the opportunity to coalesce with each other. Suitably the microdroplets employed have a finite diameter less than 100 microns, preferably less than 50 microns, more preferably less than 20 microns and even more preferably less than 15 microns. Most preferably of all their diameters are in the range 2 to 20 microns. In one embodiment, the microdroplet flow rate through the whole system is in the range 50 to 3000 microdroplets per second preferably 100 to 2000.

In a second aspect of the present invention there is provided a multi-component biological probe system characterised by including (1) a primary probe comprising (a) a first single-stranded oligonucleotide including a restriction endonuclease nicking-site, a single nucleotide capture site for capturing a single nucleoside triphosphate and oligonucleotide flanking regions juxtaposed either side of the capture site and (b) second and third single-stranded oligonucleotides capable of hybridising to the flanking regions and (2) a secondary probe comprising (c) an at least partially single-stranded fourth oligonucleotide bearing fluorophores in a substantially undetectable state having a single-stranded region complementary to at least part of the first oligonucleotide and (d) optionally a single-stranded fifth oligonucleotide at least in part complementary to the fourth oligonucleotide.

In one embodiment the second and third oligonucleotides are connected by a linker region; for example, an oligonucleotide region. In another the nicking site is an oligonucleotide region including the capture site. In yet another embodiment the fourth oligonucleotide is either (1) partially double-stranded, i.e. j-shaped, or (2) single-stranded and used in association with the fifth oligonucleotide.

Suitably, the primary probe comprises from one to four different first oligonucleotide types differing in the sequences of their flanking regions and in the nucleotide characteristic of the capture region. In another embodiment the secondary probe comprises from one to four different fourth oligonucleotide types differing in the fluorophores they bear and in their sequences.

Preferably the probe system further comprises at least one of a ligase, a polymerase, an exonuclease having double-stranded exonuclease activity, and a nicking restriction endonuclease capable of nicking the nicking site once the primary probe has been used.

Details of suitable nicking restriction endonucleases which can be used with the method, primary probes and probe systems of the present invention can be found at http://rebase.neb.com in the database associated therewith.

It is believed that the detection method used in the sequencing method described above is also generally applicable to the analysis, characterisation or quantification of single nucleoside triphosphates in a biological sample or an analyte derived therefrom. Thus, in a third aspect of the present invention there is provided a method of analysing a single nucleoside triphosphate characterised by the steps of (1) producing at least one substantially double-stranded primary oligonucleotide used probe by reacting, in the presence of a polymerase and a ligase, the single nucleoside triphosphate with a corresponding primary probe comprising (a) a first single-stranded oligonucleotide including a restriction endonuclease nicking-site, a single nucleotide capture site for capturing the single nucleoside triphosphate and oligonucleotide flanking regions juxtaposed either side of the capture site and (b) second and third single-stranded oligonucleotides capable of hybridising to the first oligonucleotide flanking regions; (2) nicking the first oligonucleotide strand of the used primary probe at the nicking-site with a nicking restriction endonuclease to create separate first oligonucleotide components; (3) separating the first oligonucleotide components generated in step (2) from the complementary strand of the used probe; (4) producing at least one substantially double-stranded secondary used probe by reacting, in the presence of a ligase, at least one of the separated first oligonucleotide components with a corresponding secondary probe comprising (c) a complementary fourth oligonucleotide bearing fluorophores in a substantially undetectable state and optionally (d) a single-stranded fifth oligonucleotide at least in part complementary to the fourth oligonucleotide; (5) digesting the used secondary probe with an enzyme having double-stranded exonucleolytic activity to yield the fluorophores in a detectable state and a single-stranded sixth oligonucleotide which is at least in part the sequence complement of the fourth oligonucleotide and (6) detecting the fluorophores released in step (5).

In such a method the nature of the various steps and the biological probes employed will suitably be as described above. In one embodiment the single nucleoside triphosphate will be derived from a precursor double-stranded DNA analyte by pyrophosphorolysis. In another it will be generated from a precursor single nucleoside monophosphate or single nucleoside diphosphate using for example a kinase (see for example Biotechnology and Bioengineering by Bao and Ryu (DOI 10.1002/bit.21498)).

The invention is now illustrated with reference to the following Examples.

### Example 1 - Preparation and use of a probe system

A single-stranded first oligonucleotide 1 was prepared, having the following nucleotide sequence:
5'-TCCGTGAGTCCCACAAACCAATAACCTCGTCATCATTGCACATACGCTTTGACA/InvdT/-3' wherein A, C, G, and T represent nucleotides bearing the relevant characteristic nucleotide base of DNA. It further comprises a capture region (C nucleotide) at the 34^{th} base from its 5' end, selective for capturing deoxyguanosine triphosphate nucleotides (dGTPs) in a mixture of deoxynucleoside triphosphates (dNTPs), and the recognition sequence for the nicking endonuclease Nb.BsrDI, 'NNCATTGC'.

Another single-stranded oligonucleotide 2, comprising an oligonucleotide region having a sequence complementary to the 3' flanking region of the first oligonucleotide, and a single-stranded oligonucleotide 3, comprising an oligonucleotide region having a sequence complementary to the 5' flanking region of the first oligonucleotide with a two base mismatch, a 5' phosphate group and a 3' inverted deoxythymidine nucleotide, were also prepared. They had the following nucleotide sequences:
Oligonucleotide 2: 5'-CGTATGTGCAAT-3'
Oligonucleotide 3: 5'-PATGACGAGGTTAAA/InvdT/-3'
wherein P represents the 5' phosphate group and /InvdT/ the inverted deoxythymidine nucleotide.

A single-stranded fourth oligonucleotide was also prepared, having the following nucleotide sequence:
5'-TCGTGCCTCATCGAACATGACGAGGXXQXXGGTTTGTGGT-3'
wherein X represents a deoxythymidine nucleotide (T) labelled with Atto 655 dye using conventional amine attachment chemistry and Q represents a deoxythymidine nucleotide labelled with a BHQ-2 quencher. It further comprises a 3' region complementary to the 5' flanking region of the first oligonucleotide.

A single-stranded fifth oligonucleotide was also prepared, having the following nucleotide sequence, which is substantially complementary to the 5' region of the fourth oligonucleotide:
5'-PGTTCGATGAGGCACCTTAGATGTACG/InvdT/-3'

A reaction mixture comprising the probe system was then prepared. It had a composition corresponding to that derived from the following formulation:
20uL 5x buffer pH 7.6
4uL oligonucleotide 1, 1000nM
5uL oligonucleotide 2, 100nM
6uL oligonucleotide 3, 1000nM
2uL oligonucleotide 4, 1000nM
5uL oligonucleotide 5, 1000nM
10U Nb.BsrDI nicking endonuclease (ex. New England Biolabs Inc.)
7.1U Taq Ligase
7.1U E. Coli Ligase
5.8U Bst Large Fragment polymerase
1.4U Thermostable Inorganic Pyrophosphatase
0.21 reactions PfuUltra II Fusion HS polymerase
1.25uL mixture of dNTPs, 1 nM
Water to 100uL
wherein the 5x buffer comprised the following mixture:
60uL Tris-HCl buffer, 1M, pH 7.6
25uL aqueous Magnesium Chloride, 1M
100uL aqueous Potassium Chloride, 1M
5uL Spermine solution, 1M
5uL Spermidine solution, 1M
7.5uLTetraethylenepentamine solution, 10%
50uL Triton X-100 surfactant (10%)
2.5uL Dithiothreitol solution, 1M
20uL Nicotinamide adenine dinucleotide solution, 100µM
Water to 1mL

Capture of the dGTPs and ligation of oligonucleotide 2 to oligonucleotide 3 to form a used primary probe was then carried out by incubating the mixture at 37°C for 10 minutes after which the temperature was increased to 50°C for a further 30 minutes to allow iterated nicking of the first oligonucleotide and ligation of the resulting first oligonucleotide components to the fifth oligonucleotide against the fourth oligonucleotide to form completed secondary probes. The temperature was then increased to 70°C to activate the PfuUltra II Fusion HS polymerase, allowing digestion of the fourth oligonucleotide component of the completed secondary probes to occur in an iterated cycle. The reaction mixture was illuminated using the 633nm line of a Helium-Neon laser and the resulting characteristic fluorescence of the Atto 655 dye detected using a camera as the cycles of endonucleolysis and exonucleolysis occurred.

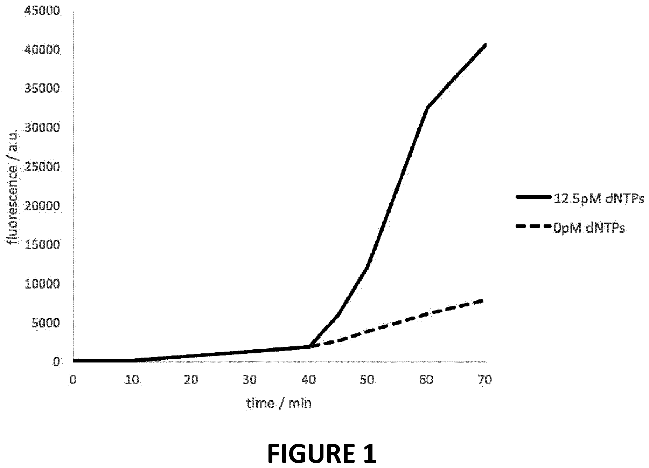

The growth in intensity of fluorescence over time in the presence and absence of the dNTP component of the reaction was monitored and the results shown graphically in figure 1. From this it can be seen that the probe system efficiently captures the dGTPs and the cyclic nature of the process of the present invention leads to a rapid growth in fluorescence signal. On the contrary, in a comparative experiment where no dNTPs were present in the reaction mixture the Atto 655 dye on oligonucleotide 4 did not exhibit fluorescence to any significant extent.

### Example 2 - Droplet microfluidic method using the probe system

Figure 2 schematically illustrates a microfluidic sequencing device in which microdroplets each containing a single nucleotide base are made to undergo reaction with a probe system of the type above as described above.

An aqueous medium 1 comprising a stream of single nucleotide triphosphates obtained by the progressive pyrophosphorolysis of a 100 nucleotide base polynucleotide analyte derived from human DNA is caused to flow through a ten-micron diameter microfluidic tube fabricated from PDMS polymer. The pyrophosphorolysis reaction itself is carried out by passing a stream of an aqueous, buffered (pH 7.5) reaction medium at 72°C, comprising *Taq Pol* and a solution having a 2 millimoles per litre concentration of each of sodium pyrophosphate and magnesium chloride, over a glass micro bead onto which the analyte has been previously attached by means of a succinyl bridge. The order of the single nucleotides in 1, which is downstream of the micro bead, corresponds to the sequence of the analyte. 1 emerges from a droplet head 2 into a first chamber 3 where it is contacted with one or more streams of immiscible light silicone oil 4. The velocities of these streams are chosen to avoid turbulent mixing and to create aqueous spherical droplets 5 suspended in the oil each having a diameter of approximately eight microns. Typically, rates are adjusted so that between adjacent filled droplets there are on average 10 empty ones. A stream of 5 is then carried forward along a second microfluidic tube of the same diameter to a second chamber 6 into which a second stream of five micron aqueous spherical droplets 7 is also fed by means of a second droplet head 8. Droplets 5 and 7 are caused to coalesce in a sequential fashion to form enlarged aqueous droplets 9 approximately nine microns in diameter. Each of 7 contains inorganic pyrophosphatase to destroy any residual pyrophosphate anion present in each of 5.

A stream of 9 is then carried forward at the same rate via microfluidic tubing into a third chamber 10 where these droplets are contacted with a third stream of five micron aqueous spherical droplets 11 also fed thereto through a corresponding droplet head 12. The time taken for each of 9 to move between chambers 6 and 10 is c.2minutes.

Droplets 9 and 11 are then caused to coalesce in 10 to produce droplets 13 (approximately ten microns in diameter). Each of 11 contains a mesophilic ligase, a thermophilic polymerase, the nicking restriction endonuclease Nb.BsrDI (ex. New England Biolabs Inc.), a primary probe comprising four sets of single-stranded oligonucleotides similar to those described in Example 1, a secondary probe comprising four different fourth single-stranded oligonucleotide labelled with a different fluorophore and four complementary fifth oligonucleotides and exonuclease. Alternatively, these components may be added in a series of coalescence steps (not shown) in which different droplet types 11, each containing one or more of these components, are coalesced in turn with 9 or a droplet arising from a previous coalescence to eventually yield 13.

The stream of the coalesced microdroplets 13 so formed is then subjected to incubation at 37°C for 10 minutes followed by 50°C for 30 minutes followed by 70°C for 30 minutes. At the end of this time 13 is transferred to the detection system, 14.

The detection system (not shown) typically comprises a detection window in which each droplet is interrogated with incident light from a laser. Action of this light then causes the released fluorophores in each droplet to fluoresce in a way characteristic of the single nucleotide base which was originally incorporated into the captured molecule (or essentially not at all if the droplet was originally empty). The presence or absence of this fluorescence is then detected at the four characteristic wavelengths of the four fluorophores associated with the four oligonucleotide sets mentioned above. Thus as the droplets are interrogated in turn the sequence of nucleotide bases in the original polynucleotide analyte can in effect be read off.

## Claims

1. A method of sequencing a nucleic acid **characterised by** the steps of (1) generating a stream of single nucleoside triphosphates by progressive enzymatic digestion of the nucleic acid; (2) producing at least one substantially double-stranded primary oligonucleotide used probe by reacting, in the presence of a polymerase and a ligase, at least one of the single nucleoside triphosphates with a corresponding primary probe comprising (a) a first single-stranded oligonucleotide including a single nucleotide capture site for capturing the single nucleoside triphosphate, a restriction endonuclease nicking site including the capture site and oligonucleotide flanking regions juxtaposed either side of the capture site and (b) second and third single-stranded oligonucleotides capable of hybridising to the first oligonucleotide flanking regions; (3) nicking the first oligonucleotide strand of the used primary probe at the nicking-site with a nicking restriction endonuclease to create separate first oligonucleotide components; (4) separating the first oligonucleotide components generated in step (3) from the complementary strand of the used probe; (5) producing at least one substantially double-stranded secondary used probe by reacting, in the presence of a ligase, at least one of the separated first oligonucleotide components with a corresponding secondary probe comprising (c) a fourth oligonucleotide having an oligonucleotide region which is complementary to a corresponding region of the first oligonucleotide component and bearing fluorophores in a substantially undetectable state and optionally (d) a fifth oligonucleotide having an oligonucleotide region which is at least in part complementary to another corresponding region of the fourth oligonucleotide to produce a double-stranded used secondary probe comprised of the first oligonucleotide component, the fourth oligonucleotide and optionally the fifth oligonucleotide; (6) digesting the fourth oligonucleotide strand of the used secondary probe with an enzyme having double-stranded exonucleolytic activity to yield single nucleotides bearing fluorophores in a detectable state and (7) detecting the fluorophores released in step (6).

2. A method as claimed in claim 1 **characterised in that** the complementary strand of the used probe released in step (4) is reacted with a further molecule of the first oligonucleotide and steps (3) and (4) are thus iterated in a first cycle.

3. A method as claimed in claim 1 or claim 2 **characterised in that** the sixth oligonucleotide generated in step (6) is reacted with a further molecule of the fourth oligonucleotide and step (6) thereafter repeated to create a second cycle.

4. A method as claimed in any of claims 1 to 3 **characterised in that** at least one of the first oligonucleotide components further comprises fluorophores arranged so that the first oligonucleotide is substantially non-fluorescing.

5. A method as claimed in claim 4 **characterised in that** at least one of the first oligonucleotide components further comprises quenchers.

6. A method as claimed in any of the preceding claims **characterised in that** the second oligonucleotide and the third oligonucleotide are connected by a linker region.

7. A method as claimed in claim 6 **characterised in that** the linker region comprises an oligonucleotide region.

8. A method as claimed in claim 6 or 7 **characterised in that** the complementary strand of the used primary probe comprises a closed-loop.

9. A method as claimed in any of the preceding claims **characterised in that** the nicking restriction endonuclease is a conventional restriction endonuclease and the complementary strand of the used primary probe is rendered resistant to endonucleolysis.

10. A method as claimed in any of the preceding claims **characterised in that** either or both of the first oligonucleotide components, the third oligonucleotide, and the fifth oligonucleotide include an element resistant to exonucleolytic degradation.

11. A method as claimed in any of the preceding claims **characterised in that** up to four different secondary probe types are employed, the fourth oligonucleotide of each having different fluorophores and a region complementary to a different first oligonucleotide component.

12. A method as claimed in any of the preceding claims **characterised in that** up to four different primary probe types are employed, the first oligonucleotide of each having a capture site selective for one of the characteristic nucleobases of naturally-occurring DNA or RNA and optionally different fluorophores.

13. A method as claimed in any of the preceding claims **characterised in that** step (1) further comprises containing each single nucleoside triphosphate in a corresponding microdroplet and that steps (2) to (7) are carried out on each microdroplet.

14. A multi-component biological probe system **characterised by** including (1) a primary probe comprising (a) a first single-stranded oligonucleotide including a single nucleotide capture site for capturing a single nucleoside triphosphate, a restriction endonuclease nicking site including the capture site and oligonucleotide flanking regions juxtaposed either side of the capture site and (b) second and third single- stranded oligonucleotides capable of hybridising to the flanking regions and (2) a secondary probe comprising (c) an at least partially single-stranded fourth oligonucleotide having an oligonucleotide region which is complementary to a corresponding region of the first oligonucleotide component and bearing fluorophores in a substantially undetectable state and (d) optionally a single-stranded fifth oligonucleotide having an oligonucleotide region which is complementary to another corresponding region of the fourth oligonucleotide.

15. A method of analysing a single nucleoside triphosphate **characterised by** the steps of (1) producing at least one substantially double-stranded primary oligonucleotide used probe by reacting, in the presence of a polymerase and a ligase, the single nucleoside triphosphate with a corresponding primary probe comprising (a) a first single-stranded oligonucleotide including a single nucleotide capture site for capturing the single nucleoside triphosphate, a restriction endonuclease nicking site including the capture site and oligonucleotide flanking regions juxtaposed either side of the capture site and (b) second and third single-stranded oligonucleotides capable of hybridising to the first oligonucleotide flanking regions; (2) nicking the first oligonucleotide strand of the used primary probe at the nicking-site with a nicking restriction endonuclease to create separate first oligonucleotide components; (3) separating the first oligonucleotide components generated in step (2) from the complementary strand of the used probe; (4) producing at least one substantially double-stranded secondary used probe by reacting, in the presence of a ligase, at least one of the separated first oligonucleotide components with a corresponding secondary probe comprising (c) a fourth oligonucleotide having an oligonucleotide region which is complementary to a corresponding region on the first oligonucleotide component and bearing fluorophores in a substantially undetectable state and optionally (d) a single-stranded fifth oligonucleotide having an oligonucleotide region which is complementary to another corresponding region on the fourth oligonucleotide to produce a double-stranded used secondary probe comprised of the first oligonucleotide component, the fourth oligonucleotide and optionally the fifth oligonucleotide; (5) digesting the fourth oligonucleotide strand of the used secondary probe with an enzyme having double-stranded exonucleolytic activity to yield single nucleotides bearing the fluorophores in a detectable state and (6) detecting the fluorophores released in step (5).

## Patentansprüche

1. Verfahren zur Sequenzierung einer Nukleinsäure, **gekennzeichnet durch** die Schritte (1) Erzeugen eines Stroms von Einzelnukleosidtriphosphaten durch progressive enzymatische Verdauung der Nukleinsäure; (2) Herstellen wenigstens einer weitgehend doppelsträngigen primären verwendeten Oligonukleotidsonde durch Umsetzen wenigstens eines der Einzelnukleosidtriphosphate in Gegenwart einer Polymerase und einer Ligase mit einer entsprechenden primären Sonde, die (a) ein erstes einzelsträngiges Oligonukleotid, das eine Einzelnukleotid-Einfangstelle zum Einfangen des Einzelnukleosidtriphosphats, eine Restriktionsendonuklease-Nicking-Stelle, die die Einfangstelle enthält, und flankierende Oligonukleotidregionen, die jeweils neben beiden Seiten der Einfangstelle gelegen sind, enthält, und (b) zweite und dritte einzelsträngige Oligonukleotide, die an die flankierenden Regionen des ersten Oligonukleotids hybridisieren können, umfasst; (3) Nicking des Erstes-Oligonukleotid-Strangs der verwendeten primären Sonde an der Nicking-Stelle mit einer Nicking-Restriktionsendonuklease, so dass getrennte Komponenten des ersten Oligonukleotids entstehen; (4) Trennen der in Schritt (3) erzeugten Komponenten des ersten Oligonukleotids vom Komplementärstrang der verwendeten Sonde; (5) Herstellen wenigstens einer weitgehend doppelsträngigen sekundären verwendeten Sonde durch Umsetzen wenigstens einer der getrennten Komponenten des ersten Oligonukleotids in Gegenwart einer Ligase mit einer entsprechenden sekundären Sonde, die (c) ein viertes Oligonukleotid, das eine Oligonukleotidregion, die zu einer entsprechenden Region der Komponente des ersten Oligonukleotids komplementär ist, aufweist und Fluorophore in einem weitgehend nicht nachweisbaren Zustand trägt, und gegebenenfalls (d) ein fünftes Oligonukleotid mit einer Oligonukleotidregion, die wenigstens zum Teil zu einer weiteren entsprechenden Region des vierten Oligonukleotids komplementär ist, umfasst, so dass eine doppelsträngige verwendete sekundäre Sonde produziert wird, die aus der Komponente des ersten Oligonukleotids, dem vierten Oligonukleotid und gegebenenfalls dem fünften Oligonukleotid besteht; (6) Verdauen des Viertes-Oligonukleotid-Strangs der verwendeten sekundären Sonde mit einem Enzym mit Doppelstrang-Exonukleolyse-Aktivität, so dass Fluorophore in einem nachweisbaren Zustand tragende Einzelnukleotide erhalten werden, und (7) Nachweisen der in Schritt (6) freigesetzten Fluorophore.

2. Verfahren nach Anspruch 1, **dadurch gekennzeichnet, dass** der in Schritt (4) freigesetzte Komplementärstrang der verwendeten Sonde mit einem weiteren Molekül des ersten Oligonukleotids umgesetzt wird und Schritt (3) und (4) somit in einem ersten Zyklus iteriert werden.

3. Verfahren nach Anspruch 1 oder Anspruch 2, **dadurch gekennzeichnet, dass** das in Schritt (6) erzeugte sechste Oligonukleotid mit einem weiteren Molekül des vierten Oligonukleotids umgesetzt wird und Schritt (6) danach wiederholt wird, so dass ein zweiter Zyklus entsteht.

4. Verfahren nach einem der Ansprüche 1 bis 3, **dadurch gekennzeichnet, dass** wenigstens eine der Komponenten des ersten Oligonukleotids ferner Fluorophore umfasst, die so angeordnet sind, dass das erste Oligonukleotid weitgehend nicht fluoreszierend ist.

5. Verfahren nach Anspruch 4, **dadurch gekennzeichnet, dass** wenigstens eine der Komponenten des ersten Oligonukleotids ferner Quencher umfasst.

6. Verfahren nach einem der vorhergehenden Ansprüche, **dadurch gekennzeichnet, dass** das zweite Oligonukleotid und das dritte Oligonukleotid durch eine Linkerregion verbunden sind.

7. Verfahren nach Anspruch 6, **dadurch gekennzeichnet, dass** die Linkerregion eine Oligonukleotidregion umfasst.

8. Verfahren nach Anspruch 6 oder 7, **dadurch gekennzeichnet, dass** der Komplementärstrang der verwendeten primären Sonde eine Closed-loop umfasst.

9. Verfahren nach einem der vorhergehenden Ansprüche, **dadurch gekennzeichnet, dass** es sich bei der Nicking-Restriktionsendonuklease um eine herkömmliche Restriktionsendonuklease handelt und der Komplementärstrang der verwendeten primären Sonde resistent gegen Endonukleolyse gemacht wird.

10. Verfahren nach einem der vorhergehenden Ansprüche, **dadurch gekennzeichnet, dass** eine der oder beide Komponenten des ersten Oligonukleotids, das dritte Oligonukleotid und das fünfte Oligonukleotid ein Element enthalten, das resistent gegen exonukleolytischem Abbau ist.

11. Verfahren nach einem der vorhergehenden Ansprüche, **dadurch gekennzeichnet, dass** bis zu vier verschiedene Sekundäre-Sonde-Typen eingesetzt werden, bei denen jeweils das vierte Oligonukleotid unterschiedliche Fluorophore und eine zu einer unterschiedlichen Komponente des ersten Oligonukleotids komplementäre Region aufweist.

12. Verfahren nach einem der vorhergehenden Ansprüche, **dadurch gekennzeichnet, dass** bis zu vier verschiedene Primäre-Sonde-Typen eingesetzt werden, bei denen jeweils das erste Oligonukleotid eine Einfangstelle, die für eine der charakteristischen Nukleobasen natürlich vorkommender DNA oder RNA selektiv ist, und gegebenenfalls unterschiedliche Fluorophore aufweist.

13. Verfahren nach einem der vorhergehenden Ansprüche, **dadurch gekennzeichnet, dass** Schritt (1) ferner umfasst, dass jedes Einzelnukleosidtriphosphat in einem entsprechenden Mikrotröpfchen enthalten ist und dass die Schritte (2) bis (7) bei jedem Mikrotröpfchen durchgeführt werden.

14. Biologisches Mehrkomponenten-Sondensystem, **dadurch gekennzeichnet, dass** es (1) eine primäre Sonde, die (a) ein erstes einzelsträngiges Oligonukleotid, das eine Einzelnukleotid-Einfangstelle zum Einfangen eines Einzelnukleosidtriphosphats, eine Restriktionsendonuklease-Nicking-Stelle, die die Einfangstelle enthält, und flankierende Oligonukleotidregionen, die jeweils neben beiden Seiten der Einfangstelle gelegen sind, enthält, und (b) zweite und dritte einzelsträngige Oligonukleotide, die an die flankierenden Regionen hybridisieren können, umfasst, und (2) eine sekundäre Sonde, die (c) ein wenigstens teilweise einzelsträngiges viertes Oligonukleotid, das eine Oligonukleotidregion, die zu einer entsprechenden Region der Komponente des ersten Oligonukleotids komplementär ist, aufweist und Fluorophore in einem weitgehend nicht nachweisbaren Zustand trägt, und (d) gegebenenfalls ein einzelsträngiges fünftes Oligonukleotid mit einer Oligonukleotidregion, die zu einer weiteren entsprechenden Region des vierten Oligonukleotids komplementär ist, umfasst, enthält.

15. Verfahren zur Analyse eines Einzelnukleosidtriphosphats, **gekennzeichnet durch** die Schritte (1) Herstellen wenigstens einer weitgehend doppelsträngigen primären verwendeten Oligonukleotidsonde durch Umsetzen des Einzelnukleosidtriphosphats in Gegenwart einer Polymerase und einer Ligase mit einer entsprechenden primären Sonde, die (a) ein erstes einzelsträngiges Oligonukleotid, das eine Einzelnukleotid-Einfangstelle zum Einfangen des Einzelnukleosidtriphosphats, eine Restriktionsendonuklease-Nicking-Stelle, die die Einfangstelle enthält, und flankierende Oligonukleotidregionen, die jeweils neben beiden Seiten der Einfangstelle gelegen sind, enthält, und (b) zweite und dritte einzelsträngige Oligonukleotide, die an die flankierenden Regionen des ersten Oligonukleotids hybridisieren können, umfasst; (2) Nicking des Erstes-Oligonukleotid-Strangs der verwendeten primären Sonde an der Nicking-Stelle mit einer Nicking-Restriktionsendonuklease, so dass getrennte Komponenten des ersten Oligonukleotids entstehen; (3) Trennen der in Schritt (2) erzeugten Komponenten des ersten Oligonukleotids vom Komplementärstrang der verwendeten Sonde; (4) Herstellen wenigstens einer weitgehend doppelsträngigen sekundären verwendeten Sonde durch Umsetzen wenigstens einer der getrennten Komponenten des ersten Oligonukleotids in Gegenwart einer Ligase mit einer entsprechenden sekundären Sonde, die (c) ein viertes Oligonukleotid, das eine Oligonukleotidregion, die zu einer entsprechenden Region auf der Komponente des ersten Oligonukleotids komplementär ist, aufweist und Fluorophore in einem weitgehend nicht nachweisbaren Zustand trägt, und gegebenenfalls (d) ein einzelsträngiges fünftes Oligonukleotid mit einer Oligonukleotidregion, die wenigstens zum Teil zu einer weiteren entsprechenden Region auf dem vierten Oligonukleotids komplementär ist, umfasst, so dass eine doppelsträngige verwendete sekundäre Sonde produziert wird, die aus der Komponente des ersten Oligonukleotids, dem vierten Oligonukleotid und gegebenenfalls dem fünften Oligonukleotid besteht; (5) Verdauen des Viertes-Oligonukleotid-Strangs der verwendeten sekundären Sonde mit einem Enzym mit Doppelstrang-Exonukleolyse-Aktivität, so dass die Fluorophore in einem nachweisbaren Zustand tragende Einzelnukleotide erhalten werden, und (6) Nachweisen der in Schritt (5) freigesetzten Fluorophore.

## Revendications

1. Procédé de séquençage d'un acide nucléique **caractérisé par** les étapes de (1) génération d'un flux de nucléoside triphosphate uniques par digestion enzymatique progressive de l'acide nucléique ; (2) production d'au moins une sonde oligonucléotidique primaire sensiblement double brin utilisée par réaction, en présence d'une polymérase et d'une ligase, au moins un des nucléosides triphosphates uniques avec une sonde primaire correspondante comprenant (a) un premier oligonucléotide simple brin comprenant un site de capture d'un seul nucléotide pour capturer le nucléoside triphosphate unique, un site de coupure simple brin d'endonucléase de restriction comprenant le site de capture et des régions flanquantes oligonucléotidiques juxtaposées de chaque côté du site de capture et (b) des deuxième et troisième oligonucléotides simples brins capables de s'hybrider avec les premières régions flanquantes oligonucléotidiques ; (3) coupure simple brin du premier brin oligonucléotidique de la sonde primaire utilisée au niveau du site de coupure simple brin avec une endonucléase de restriction de coupure simple brin pour créer des composants de premier oligonucléotide séparés ; (4) séparation des composants de premier oligonucléotide générés dans l'étape (3) du brin complémentaire de la sonde utilisée ; (5) production d'au moins une sonde secondaire sensiblement double brin utilisée par réaction, en présence d'une ligase, d'au moins un des composants de premier oligonucléotide séparés avec une sonde secondaire correspondante comprenant (c) un quatrième oligonucléotide comportant une région oligonucléotidique qui est complémentaire d'une région correspondante sur le composant de premier oligonucléotide et portant des fluorophores dans un état sensiblement indétectable et facultativement (d) un cinquième oligonucléotide comportant une région oligonucléotidique qui est, au moins en partie, complémentaire d'une autre région correspondante du quatrième oligonucléotide pour produire une sonde secondaire double brin utilisée constituée du composant de premier oligonucléotide, du quatrième oligonucléotide et facultativement du cinquième oligonucléotide ; (6) digestion du quatrième brin oligonucléotidique de la sonde secondaire utilisée avec une enzyme ayant une activité exonucléolytique double brin pour obtenir des nucléotides uniques portant des fluorophores dans un état détectable et (7) détection des fluorophores libérés dans l'étape (6).

2. Procédé selon la revendication 1, **caractérisé en ce que** le brin complémentaire de la sonde utilisée libéré dans l'étape (4) réagit avec une molécule supplémentaire du premier oligonucléotide et les étapes (3) et (4) sont ainsi répétées dans un premier cycle.

3. Procédé selon la revendication 1 ou la revendication 2 **caractérisé en ce que** le sixième oligonucléotide généré dans l'étape (6) réagit avec une molécule supplémentaire du quatrième oligonucléotide et l'étape (6) est ensuite répétée pour créer un deuxième cycle.

4. Procédé selon l'une quelconque des revendications 1 à 3, **caractérisé en ce qu'**au moins un des composants de premier oligonucléotide comprend en outre des fluorophores agencés de sorte que le premier oligonucléotide est sensiblement non fluorescent.

5. Procédé selon la revendication 4, **caractérisé en ce qu'**au moins un des composants de premier oligonucléotide comprend en outre des désactiveurs.

6. Procédé selon l'une quelconque des revendications précédentes, **caractérisé en ce que** le deuxième oligonucléotide et le troisième oligonucléotide sont reliés par une région de lieur.

7. Procédé selon la revendication 6, **caractérisé en ce que** la région de lieur comprend une région oligonucléotidique.

8. Procédé selon la revendication 6 ou 7, **caractérisé en ce que** le brin complémentaire de la sonde primaire utilisée comprend une boucle fermée.

9. Procédé selon l'une quelconque des revendications précédentes, **caractérisé en ce que** l'endonucléase de restriction de coupure simple brin est une endonucléase de restriction conventionnelle et le brin complémentaire de la sonde primaire utilisée est rendue résistante à l'endonucléolyse.

10. Procédé selon l'une quelconque des revendications précédentes, **caractérisé en ce que** l'un ou les deux parmi les composants de premier oligonucléotide, le troisième oligonucléotide et le cinquième oligonucléotide comprennent un élément résistant à la dégradation exonucléolytique.

11. Procédé selon l'une quelconque des revendications précédentes, **caractérisé en ce que** jusqu'à quatre types de sonde secondaire différents sont utilisés, le quatrième oligonucléotide de chacun d'entre eux ayant différents fluorophores et une région complémentaire d'un composant de premier oligonucléotide différent.

12. Procédé selon l'une quelconque des revendications précédentes **caractérisé en ce que** jusqu'à quatre types de sonde primaire différent sont utilisés, le premier oligonucléotide de chacun d'entre eux comportant un site de capture sélectif pour l'une des bases azotées caractéristiques d'ADN ou d'ARN d'origine naturelle et facultativement différents fluorophores.

13. Procédé selon l'une quelconque des revendications précédentes, **caractérisé en ce que** l'étape (1) comprend en outre l'inclusion de chaque nucléoside triphosphate unique dans une microgouttelette correspondante et que les étapes (2) à (7) sont conduites sur chaque microgouttelette.

14. Système de sonde biologique multicomposant **caractérisé en ce qu'**il comprend (1) une sonde primaire comprenant (a) un premier oligonucléotide simple brin comprenant un site de capture d'un seul nucléotide pour capturer un nucléoside triphosphate unique, un site de coupure simple brin d'endonucléase de restriction comprenant le site de capture et des régions flanquantes oligonucléotidiques juxtaposées de chaque côté du site de capture et (b) des deuxième et troisième oligonucléotides simples brins capables de s'hybrider avec les premières régions flanquantes et (2) une sonde secondaire comprenant (c) un quatrième oligonucléotide au moins partiellement simple brin comportant une région oligonucléotidique qui est complémentaire d'une région correspondante du composant de premier oligonucléotide et portant des fluorophores dans un état sensiblement indétectable et (d) facultativement, un cinquième oligonucléotide simple brin comportant une région oligonucléotidique qui est complémentaire d'une autre région correspondante du quatrième oligonucléotide.

15. Procédé d'analyse d'un nucléoside triphosphate unique **caractérisé par** les étapes de (1) production d'au moins une sonde oligonucléotidique primaire sensiblement double brin utilisée par réaction, en présence d'une polymérase et d'une ligase, du nucléoside triphosphate unique avec une sonde primaire correspondante comprenant (a) un premier oligonucléotide simple brin comprenant un site de capture d'un seul nucléotide pour capturer le nucléoside triphosphate unique, un site de coupure simple brin d'endonucléase de restriction comprenant le site de capture et des régions flanquantes oligonucléotidiques juxtaposées de chaque côté du site de capture et (b) des deuxième et troisième oligonucléotides simples brins capables de s'hybrider avec les premières régions flanquantes oligonucléotidiques ; (2) coupure simple brin du premier brin oligonucléotidique de la sonde primaire utilisée au niveau du site de coupure simple brin avec une endonucléase de restriction de coupure simple brin pour créer des composants de premier oligonucléotide séparés ; (3) séparation des composants de premier oligonucléotide générés dans l'étape (2) du brin complémentaire de la sonde utilisée ; (4) production d'au moins une sonde secondaire sensiblement double brin utilisée par réaction, en présence d'une ligase, au moins un des composants de premier oligonucléotide séparés avec une sonde secondaire correspondante comprenant (c) un quatrième oligonucléotide comportant une région oligonucléotidique qui est complémentaire d'une région correspondante sur le composant de premier oligonucléotide et portant des fluorophores dans un état sensiblement indétectable et facultativement (d) un cinquième oligonucléotide simple brin comportant une région oligonucléotidique qui est complémentaire d'une autre région correspondante sur le quatrième oligonucléotide pour produire une sonde secondaire double brin utilisée constituée du composant de premier oligonucléotide, du quatrième oligonucléotide et facultativement du cinquième oligonucléotide ; (5) digestion du quatrième brin oligonucléotidique de la sonde secondaire utilisée avec une enzyme ayant une activité exonucléolytique double brin pour obtenir des nucléotides uniques portant les fluorophores dans un état détectable et (6) détection des fluorophores libérés dans l'étape (5).
